# EUROPÄISCHE PATENTSCHRIFT

(11) **EP 3 079 109 B1**
(45) Veröffentlichungstag und Bekanntmachung des Hinweises auf die Patenterteilung: **19.08.2020**
(21) Anmeldenummer: 16000750.6
(22) Anmeldetag: 31.03.2016
(51) Int. Cl.: G06Q 10/00, G01N 33/00

(54) **VERFAHREN UND VORRICHTUNG ZUM VERWALTEN VON MOBILEN GERÄTEN**
METHOD AND APPARATUS FOR MANAGING MOBILE DEVICES
PROCEDE ET DISPOSITIF DE GESTION D'APPAREILS MOBILES

(30) Priorität: 07.04.2015 DE 102015004248
(43) Veröffentlichungstag der Anmeldung: 12.10.2016
(73) Patentinhaber: Dräger Safety AG & Co. KGaA, 23560 Lübeck (DE)
(72) Erfinder: Hegewald, Jan, 40477 Düsseldorf (DE); Hammer, Thielo, 40468 Düsseldorf (DE)
(74) Vertreter: Mildner, Volker

(56) Entgegenhaltungen:
- DE-A1-102011 119 570

## Beschreibung

Die vorliegende Erfindung betrifft eine Vorrichtung zum automatischen Verwalten von mobilen Geräten.

Mobile Geräte wie Gasmessgeräte, insbesondere tragbare Gasmessgeräte, kommen neben Industrieanlagen, Geschäftsräumen und öffentlichen Gebäuden überall dort zum Einsatz, wo Menschen und Eigentum vor Verletzungen und Beschädigungen geschützt werden müssen. In einer großtechnischen Anlage, wie einer Raffinerie, kann damit die Gaskonzentration wenigstens einer Gassubstanz überwacht werden. Die zu überwachenden Gaskonzentrationen betreffen beispielsweise die untere Explosionsgrenze oder Schwellenwerte von Gasen wie beispielsweise Kohlenmonoxid, Schwefelwasserstoff, Sauerstoff, oder andere toxische Gassubstanzen. Als untere Explosionsgrenze versteht man die Grenze, die beim Übertreten in Verbindung mit einer externen Energiequelle, wie beispielsweise ein Funke, zur Explosion führt. Unter einer toxischen Gassubstanz wird im Allgemeinen ein gas- oder dampfförmiger Gefahrenstoff bezeichnet, der bestimmte Gefährlichkeitsmerkmale oder sonstige chronisch schädigende Eigenschaften besitzt. Im Stand der Technik bekannte Gasmessgeräte besitzen im Allgemeinen eine oder mehrere Gaseintrittsöffnungen, durch welche die zu überprüfende Gassubstanz zu entsprechenden Sensoren geleitet wird, um die Gaskonzentration der jeweiligen Gassubstanzen zu messen. Wird in einem Gasmessgerät erkannt, dass die Konzentration einer toxischen Substanz des überprüften Gases einen definierten Schwellenwert überschreitet, wird ein optischer und/oder akustischer Alarm ausgelöst. Um einen zuverlässigen Betrieb solcher Gasmessgeräte zu gewährleisten, ist es in regelmäßigen Abständen eine Wartung erforderlich, also diese zu überprüfen, zu justieren und/oder zu kalibrieren. Unter einer Überprüfung wird im Wesentlichen ein einfacher Funktionstest des Gasmessgerätes verstanden. Bei einer Justierung werden regelmäßig Einstellungen vorgenommen, um den Nullpunkt und die Empfindlichkeit des Gasmessgerätes mit einem bekannten Null- bzw. Prüfgas zu überprüfen und einzustellen, ohne dass die bei der Erstinbetriebnahme festgelegten Parameter Gasart, Messbereich, Alarmschwellen und spezielle Anwendungen verändert werden. Bei einer Kalibrierung wird die Anzeige des Gasmessgerätes mit einer bekannten Prüfgaskonzentration verglichen.

Zur Überprüfung, Justierung und/oder Kalibrierung von Gasmessgeräten ist aus der DE 10 2012 210 085 B3 beispielsweise eine Teststation für ein tragbares Gasmessgerät für wenigstens ein Prüfgas mit einer Vorrichtung zum Aufnehmen des Gasmessgerätes, einer Prüfgasversorgung, einer schwenkbaren Klappe zum Abdecken wenigstens eines Bereichs mit Gaseintrittsöffnungen der freiliegenden Oberfläche eines in der Aufnahme aufgenommenen Gasmessgerätes und einer Dichtung bekannt.

Aus der DE 10 2011 119 570 A1 ist ein Verfahren zum Betreiben eines Gaskonzentrations-Überwachungssystems zum Erfassen der Gaskonzentration wenigstens einer Gassubstanz bekannt. Mobile Gasmessgeräte lassen sich auf ihre Funktionstüchtigkeit hin überprüfen. Die Gasmessgerät lassen sich in entsprechende Aufnahmen eines Prüfgeräts einlegen und dort warten und / oder kalibrieren. Die Funktionstüchtigkeit eines Gasmessgeräts wird detektiert. Eine Sicherheitskennzahl wird ermittelt. In Abhängigkeit der ermittelten Sicherheitskennzahl wird eine Anzeige auf grün, gelb oder rot gestellt. Diese Anzeige zeigt den Grad der Betriebstüchtigkeit des getesteten mobilen Gasmessgeräts an.

Ferner ist es im Stand der Technik bekannt, mehrere solcher Teststationen in einer Lagervorrichtung zusammenzuschalten, um das Warten mehrerer Gasmessgeräte gleichzeitig zu ermöglichen. Ein Nachteil solcher Lagervorrichtungen ist es jedoch, dass die einzelnen Wartungsstationen sehr teuer sind und somit zu erheblichen Kosten für derartige Lagervorrichtungen führen. Außerdem besteht bei den im Stand der Technik verfügbaren Lagervorrichtungen, die beispielsweise aus einer Mehrzahl von Dockingstationen für Gasmessgeräte bestehen, die Gefahr, dass auch nicht gewartete Gasmessgeräte an den Nutzer ausgegeben werden, da diese einfach aus der Dockingstation entnommen werden können. Es ist zwar bekannt, den Wartungszustand eines Gasmessgerätes über eine Lampe anzuzeigen, dies hindert den ungeschulten Nutzer jedoch nicht daran, ein Gasmessgerät, welches durch eine rot aufleuchtende Lampe beispielsweise als nicht gewartet angezeigt wird, aus der Dockingstation zu entwenden. Der Nutzer könnte die rot aufleuchtende Lampe nämlich beispielsweise versehentlich als Hinweis für einen Ladezustand und nicht für einen Wartungszustand wie einen Kalibrierungszustand der Gasmessgeräte interpretieren.

Es ist daher Aufgabe der vorliegenden Erfindung, die voranstehend beschriebenen Nachteile bei einer Vorrichtung zum Verwalten von mobilen Geräten, insbesondere Gasmessgeräten, zumindest teilweise zu vermeiden. Insbesondere ist es die Aufgabe der vorliegenden Erfindung, eine Vorrichtung zur automatisierten Lagerung, Ausgabe und/oder Aufnahme von mobilen Geräten bereitzustellen.

Die voranstehende Aufgabe wird gelöst durch eine Vorrichtung zum automatischen Verwalten von mobilen Geräten mit den Merkmalen des Anspruchs 1.

Weitere Merkmale der Erfindung ergeben sich aus den Unteransprüchen, der Beschreibung und den Zeichnungen. Dabei gelten Merkmale und Details, die im Zusammenhang mit den Verfahren beschrieben sind, selbstverständlich auch im Zusammenhang mit der erfindungsgemäßen Vorrichtung und jeweils umgekehrt, sodass bezüglich der Offenbarung zu den einzelnen Erfindungsaspekten stets wechselseitig Bezug genommen wird bzw. werden kann.

Erfindungsgemäß wird eine Vorrichtung zum automatischen Verwalten von mobilen Geräten in einer Lagervorrichtung mit wenigstens zwei Lagerstationen, wenigstens einer Wartungsstation, wenigstens einem Computer und wenigstens einer Erkennungsvorrichtung bereitgestellt. Die Vorrichtung ermöglicht, dass die folgenden Schritte durchgeführt werden:
- Erkennen eines Ausgabewunsches für die Ausgabe eines der mobilen Geräte aus der Lagervorrichtung,
- Bestimmen einer Gruppe von mobilen Geräten abhängig von einem Verhältnis wenigstens eines Wartungskennwerts der jeweiligen mobilen Geräte zu einem zugehörigen Wartungsschwellenwert mittels eines Controllers,
- Auswählen eines der mobilen Geräte aus der Gruppe als Ausgabegerät mittels des Controllers, und
- Ausgeben des ausgewählten Ausgabegerätes mittels des Controllers.

Mit der erfindungsgemäßen Vorrichtung können mobile Geräte vollautomatisch und autonom verwaltet werden. Insbesondere können sie automatisch gelagert und gezielt automatisch ausgegeben werden.

Erfindungsgemäß weist die Lagervorrichtung also wenigstens zwei Lagerstationen und wenigstens eine Wartungsstation auf. Die Lagerstationen sind jeweils zum Lagern von einem mobilen Gerät ausgebildet. So kann jede Lagerstation beispielsweise eine Aufnahme für ein mobiles Gerät aufweisen. Die Wartungsstation dient zum Warten von mobilen Geräten. Die oder jede Wartungsstation kann dazu jeweils ein mobiles Gerät aufnehmen. Sie kann deshalb ebenfalls eine Aufnahme für ein mobiles Gerät aufweisen. Darüber hinaus ist jede Wartungsstation zum Warten von mobilen Geräten ausgebildet. Dies gilt nicht für die Lagerstationen. Somit unterscheidet sich eine Wartungsstation von einer Lagerstation durch zusätzliche Wartungsschnittstellen und/oder -mittel zu bzw. für ein mobiles Gerät. Erst durch die erfindungsgemäße Wartungsstation kann ein mobiles Gerät gewartet werden. Insbesondere kann das jeweilige Gerät überprüft, justiert und/oder kalibriert werden. Insbesondere kann im Sinne der Erfindung ein Aufladen eines Akkus oder einer entsprechende Ladevorrichtung nicht als Warten bzw. nicht als Wartungsmittel verstanden sein.

Die erfindungsgemäße Lagervorrichtung kann beispielsweise als Lagerschrank mit einer Mehrzahl von Lagerfächern ausgebildet sein. Die mindestens zwei Lagerstationen können jeweils von einem der der Lagerfächer gebildet sein. Für die mindestens eine Wartungsstation kann ein weiteres der Lagerfächer dienen. Dabei sind in dem jeweiligen Lagerfach für eine Wartungsstation Wartungsmittel und/oder - schnittstellen vorgesehen, um die Wartung eines mobilen Geräts zu ermöglichen. Die Wartungsschnittstellen sind zur Kommunikation mit einem Controller und/oder zum Austausch von Fluiden zwischen dem jeweils zu wartenden mobilen Gerät und einer Fluidquelle dienen und/oder ausgebildet sein.

In einer weiteren Ausgestaltung können in den Lagerfächern jeweils eine Lagerstation oder eine Wartungsstation bereitgestellt werden. Die Lagervorrichtung kann aber auch ohne Lagerschrank aus einem Zusammenschluss aus den wenigstens zwei Lagerstationen und der wenigstens einen Wartungsstation ausgebildet sein. In diesem Fall sind die wenigstens zwei Lagerstationen und die wenigstens eine Wartungsstation bevorzugt als Dockingstationen ausgebildet.

Bei mobilen Geräten mit einem Akkumulator können die Lagerstationen und/oder die wenigstens eine Wartungsstation jeweils eine Ladevorrichtung aufweisen und/oder - ausbilden. So können sie jeweils auch zum Aufladen eines Akkumulators dienen. Die Ladevorrichtung dient in diesem Fall nicht zum Warten der mobilen Geräte. Ferner ist es im Sinne der vorliegenden Erfindung möglich, dass die Lagerstationen und/oder die wenigstens eine Wartungsstation jeweils als Lagerfach bzw. Lagerkammer ausgebildet sind, in welche ein mobiles Gerät oder eine Mehrzahl der mobilen Geräte eingelegt und gelagert werden können.

Unter dem Erkennen eines Ausgabewunsches für die Ausgabe eines mobilen Gerätes mittels einer Erkennungsvorrichtung ist zu verstehen, dass die Eingabe eines Ausgabewunsches eines Nutzers beispielsweise in eine Befehlsannahmeeinheit wie einen Touchscreen oder eine mit physischen Tasten bereitgestellte Nutzerschnittstelle erkannt wird.

Weiterhin ist die Bestimmung einer Gruppe von mobilen Geräten mittels eines Controllers vorgesehen. Die erfindungsgemäße Gruppe ist nicht auf eine Mehrzahl von mobilen Geräten beschränkt, sondern kann auch nur ein einziges mobiles Gerät aufweisen. So kann eine bestimmte Gruppe beispielsweise mehrere zehn oder hundert mobile Geräte oder nur ein einziges mobiles Gerät aufweisen.

Die Bestimmung der Gruppe von mobilen Geräten mittels des Controllers dient zur Aufgliederung der mobilen Geräte. So können die mobilen Geräte der bestimmten Gruppe von mobilen Geräten die gemeinsame Eigenschaft aufweisen, dass sie jeweils einen erforderlichen und im Voraus festgelegten Wartungszustand erfüllen. Die Bestimmung einer solchen Gruppe erfolgt dabei mittels des Wartungskennwerts der mobilen Geräte. Wird der Wartungskennwert eines jeweiligen mobilen Geräts in ein Verhältnis zu einem zugehörigen Wartungsschwellwert gesetzt, kann anhand des Verhältnisses bestimmt werden, ob das jeweilige Gerät der erfindungsgemäßen Gruppe angehört oder nicht. Das jeweilige mobile Gerät gehört der Gruppe an, wenn das zugehörige Verhältnis beispielsweise dazu korrespondiert, dass das jeweilige Gerät mindestens einen erforderlichen Wartungszustand aufweist. Ansonsten gehört das jeweilige mobile Gerät nicht der Gruppe an. Denn es weist - bezogen auf den vorherigen Fall - nicht den erforderlichen Wartungszustand auf.

Ein Wartungskennwert, d.h., ein erfasster oder bestimmter Wartungskennwert, ist beispielsweise ein Ladezustand eines Akkumulators eines mobilen Gerätes. Ein weiteres Beispiel für einen Wartungskennwert ist beispielsweise ein Kalibrierungszustand eines mobilen Geräts, insbesondere wenn dieses als Gasmessgerät ausgestaltet ist. Alternativ oder ergänzend kann ein Wartungskennwert zu einem Funktionszustand eines mobilen Geräts, insbesondere einer zugehörigen Komponente, korrespondieren. So kann der Wartungskennwert beispielsweise zu dem Funktionszustand eines Displays eines mobilen Geräts korrespondieren. Entsteht in diesem Fall ein Displayschaden, ändert sich der Wartungskennwert. Die ist insbesondere bei einem Gasmessgerät als ein mobiles Gerät sinnvoll. Denn der Anwender würde anderenfalls den Displayschaden in einem ungünstigen Fall erst sehr spät entdecken. Ein Wartungsschwellenwert ist beispielsweise ein im Voraus festgelegter Wartungsschwellenwert oder ein festlegbarer Wartungsschwellenwert.

Bei der Auswahl eines der mobilen Geräte aus der Gruppe kann ein mobiles Gerät beispielsweise anhand einer Rangfolge oder über einen Zufallsgenerator von einer Auswahlliste ausgewählt werden. So ist es beispielsweise möglich, dass in der bestimmten Gruppe anhand von verschiedenen Verhältnissen von jeweiligen Wartungskennwerten zu zugehörigen Wartungsschwellenwerten eine Rangfolge unter den mobilen Geräten der Gruppe festgelegt wird und immer eine bestimmte Position in einer entsprechend erstellten Auswahlliste, beispielsweise die erste Position, ausgewählt wird. Wenn die bestimmte Gruppe beispielsweise 100 mobile Geräte mit jeweils einem bestimmten Ladezustand des Akkumulators bzw. unterschiedlichen Ladezuständen der jeweiligen Akkumulatoren der mobilen Geräte aufweist, kann immer das mobile Gerät mit dem niedrigsten oder höchsten Ladezustand ausgewählt werden.

Unter einer Ausgabe eines mobilen Gerätes mittels des Controllers ist insbesondere die Freigabe, d.h., die mechanische und/oder elektronische Entriegelung einer Lagerstation oder einer Wartungsstation zur Entnahme des mobilen Gerätes durch einen Nutzer zu verstehen. Das heißt, bei einer Ausgabe hat ein Nutzer die Möglichkeit, das ausgewählte mobile Gerät ohne Hilfsmittel oder Überwindung von Hindernissen aus der Lagervorrichtung zu entnehmen. Gemäß der vorliegenden Erfindung ist es bei der Ausgabe also nicht erforderlich, dass das ausgewählte mobile Gerät bewegt wird. So kann das Ausgeben eines ausgewählten Ausgabegerätes beispielsweise darin bestehen, dass eine mechanische Verriegelung gelöst wird, durch welche das Ausgabegerät von der Lagervorrichtung entkoppelbar ist und somit mechanisch freigegeben wird. Es ist jedoch auch denkbar, dass das Ausgabegerät bei einer Ausgabe nicht nur in der Lagerstation oder Wartungsstation freigegeben wird, sondern mittels einer Transportvorrichtung, beispielsweise vollautomatisch, zum Nutzer oder zumindest in seinen Zugriffsbereich bewegt wird.

Gemäß einer bevorzugten Weiterbildung der vorliegenden Erfindung kann der wenigstens eine Wartungskennwert einen jeweiligen Funktionszustand der mobilen Geräte charakterisieren und der zu dem jeweiligen Wartungskennwert zugehörige Wartungsschwellenwert mit einer Funktionsfähigkeit der jeweiligen mobilen Geräte korrespondieren. Dadurch kann sichergestellt werden, dass an einen Nutzer nur funktionsfähige mobile Geräte ausgegeben werden. Unter einem Funktionszustand kann der mechanische und/oder elektronische Zustand einer Funktion eines mobilen Geräts oder die mechanische und/oder elektronische Beschaffenheit des mobilen Gerätes verstanden werden. So kann ein hoher Wartungskennwert beispielsweise auf eine vollständig vorhandene und/oder zur Nutzung zur Verfügung stehende Funktion des mobilen Geräts aufzeigen. Hingegen weist ein niedriger Wartungskennwert auf eine eingeschränkte Funktion des mobilen Geräts hin. Um ein Entscheidungskriterium beispielsweise zwischen einer noch für einen gewünschten Einsatz ausreichenden Funktion und einer für den Einsatz nicht mehr ausreichenden Funktion bilden zu können, ist vorzugsweise der Wartungsschellwert vorgesehen. Der Wartungsschwellwert dient also zur Kategorisierung des tatsächlichen Funktionszustands des mobilen Geräts. Der Wartungsschwellwert charakterisiert also beispielsweise einen minimalen Zustand der Funktion, die ein mobiles Gerät aufweisen sollte. Die Funktionsfähigkeit gibt also an, ob das mobile Gerät funktioniert oder nicht. Das heißt, wenn der Wartungskennwert beispielsweise angibt, dass ein mobiles Gerät tatsächlich nicht funktioniert, und der Wartungsschwellenwert einen Wert für ein Funktionieren des mobilen Gerätes angibt, wird das mobile Gerät nicht als ein mobiles Gerät der Gruppe bestimmt. Denn bei der Bestimmung der Gruppe wird das Verhältnis von dem tatsächlichen Wartungskennwert und dem Wartungsschwellwert berücksichtigt.

Außerdem ist es gemäß der vorliegenden Erfindung möglich, dass der wenigstens eine Wartungskennwert eine jeweilige Restlaufzeit der mobilen Geräte charakterisiert und der zugehörige Wartungsschwellenwert mit einer minimal erforderlichen Restlaufzeit der. jeweiligen mobilen Geräte korrespondiert. Aufgrund der analogen Ausgestaltung wird sinngemäß auf die Erläuterungen, Vorteile und bevorzugten Merkmale des vorangehenden Abschnitts Bezug genommen. Aufgrund der Charakterisierung der Restlaufzeit durch den Wartungskennwert erhält ein Nutzer nur mobile Geräte, deren jeweilige Restlaufzeit einer minimal erforderlichen Restlaufzeit entspricht. Denn bei der Bestimmung der Gruppe von mobilen Geräten werden nur jene Geräte ausgewählt, deren Restlaufzeit größer als die minimal erforderliche Restlaufzeit ist. Wenn die mobilen Geräte Gasmessgeräte sind und die minimal erforderliche Restlaufzeit mit einem Kalibrierungszustand der Gasmessgeräte korrespondiert, können dem Nutzer beispielsweise nur kalibrierte Gasmessgeräte ausgegeben werden, d.h. Gasmessgeräte, die mit Bezug auf ihren Kalibrierungszustand noch eine Restlaufzeit aufweisen, die länger als die minimal erforderliche Restlaufzeit ist. Die vorliegende Erfindung ist jedoch nicht darauf beschränkt, dass die minimale Restlaufeit mit einem Kalibrierungszustand korrespondiert. So ist es auch möglich, dass die minimale Restlaufzeit mit einem Ladezustand, einer Funktionsprüfungszeit, einem Mindesthaltbarkeitsdatum und/oder dergleichen korrespondiert. In bevorzugten Weiterbildungen können so beispielsweise nur mobile Geräte mit einem bestimmten minimalen Ladezustand des Akkumulators derselben, einer bestimmten höchstens verstrichenen Funktionsprüfungszeit und/oder einem noch nicht erreichten Mindesthaltbarkeitsdatum ausgegeben werden. Dadurch kann gewährleistet werden, dass der Nutzer nur mobile Geräte erhält, mit welchen er noch für eine bestimmte Zeit arbeiten kann bzw. darf. Gemäß dieser Weiterbildung ist es fernen möglich, dass an den Nutzer nur funktionsfähige Geräte mit einer bestimmten minimalen Restlaufzeit ausgegeben werden können. Die minimal erforderliche Restlaufzeit kann im Bereich von Stunden, Tagen, Wochen oder Monaten liegen. Bei Gasmessgeräten liegt sie bevorzugt bei einem Tag. Die minimal erforderliche Restlaufzeit hängt insbesondere von einer Annahme ab, für wie lange der Nutzer das mobile Gerät voraussichtlich verwendet bzw. wann und für wie lange das mobile Gerät ab Ausgabe aus der Lagervorrichtung zum Einsatz kommt.

Ferner kann es gemäß der vorliegenden Erfindung von Vorteil sein, wenn als Ausgabegerät das mobile Gerät durch den Controller ausgewählt wird, das die kürzeste Restlaufzeit hat. So kann erreicht werden, dass sich in der Lagervorrichtung stets mobile Geräte mit einem möglichst aktuellen Wartungszustand befinden und, eine entsprechend hohe Ausleih- bzw. Ausgaberate vorausgesetzt, keines der mobilen Geräte einen Wartungskennwert hat, welcher nicht mehr die minimal erforderliche Restlaufzeit aufweist. Durch dieses erfindungsgemäße Verfahren ist es besonders vorteilhaft möglich - die erforderliche Ausgaberate vorausgesetzt - eine autonome Lagervorrichtung zu schaffen.

Darüber hinaus ist es erfindungsgemäß möglich, dass das Ausgabegerät aus der wenigstens einen Wartungsstation ausgewählt wird, wenn die wenigstens eine Wartungsstation mit einem mobilen Gerät der Gruppe belegt ist. Dadurch kann erreicht werden, dass möglichst immer eine Wartungsstation frei für die Aufnahme von einem zu wartenden mobilen Gerät ist. Das heißt, bei einer Mehrzahl von Wartungsstationen, die alle belegt sind, ist bei der Auswahl eines Ausgabegerätes bevorzugt eines aus den Wartungsstationen auszuwählen. Bei einer Mehrzahl von Wartungsstationen, die nur teilweise mit mobilen Geräten belegt sind, ist es bevorzugt, so lange Ausgabegeräte aus den Wartungsstationen auszugeben, bis alle Wartungsstationen frei sind. Alternativ ist es aber auch möglich, Ausgabegeräte aus jeweiligen Lagerstationen auszugeben, obwohl sich noch mobile Geräte in einem Teil der Wartungsstationen befinden. Eine belegte Lagerstation oder Wartungsstation ist dabei eine Lagerstation bzw. Wartungsstation mit einem darin aufgenommenen mobilen Gerät. Eine freie Lagerstation oder Wartungsstation ist eine Lagerstation bzw. Wartungsstation ohne ein darin aufgenommenes mobiles Gerät.

Außerdem ist es gemäß der vorliegenden Erfindung von Vorteil, wenn ein Warnsignal nach außen ausgegeben wird, wenn erkannt wird, dass das Verhältnis des wenigstens einen Wartungskennwerts eines der mobilen Geräte zu dem zugehörigen Wartungsschwellenwert nicht einem Soll-Verhältnis entspricht. Dies hat den Vorteil, dass bei einer zu geringen Häufigkeit von Ausgaben und Rückgaben beispielsweise ein Techniker informiert werden kann, der zu alte bzw. nicht rechtzeitig gewartete mobile Geräte ersetzt oder von einer Lagerstation in eine Wartungsstation setzt. Das Warnsignal kann dabei akustisch, optisch oder in Form einer elektronischen Benachrichtigung ausgegeben werden. Das Signal kann bevorzugt in Form einer Benachrichtigung mit entsprechenden Anweisungen oder Zustandsinformationen über das betroffene mobile Gerät übermittelt werden. Das Soll-Verhältnis kann beispielsweise mit der minimal erforderlichen Restlaufzeit oder einer Zeitdauer korrespondieren, über welche das mobile Gerät ohne Wartung in einer Lagerstation aufgenommen sein darf. Wenn das mobile Gerät beispielsweise ein Gasmessgerät ist, ein Wartungskennwert des Gasmessgerätes "121 Tage seit der letzten Kalibrierung" ist und der Wartungsschwellenwert "höchstens 120 Tage seit der letzten Kalibrierung" voraussetzt, wäre das Gasmessgerät seit einem Tag nicht mehr kalibriert und ein Verhältnis "120/121" würde nicht mehr dem Soll-Verhältnis entsprechen, welches mit Bezug auf die Tage beispielsweise ein Ergebnis ">1" voraussetzt. In diesem Fall würde nach 121 Tagen ohne Kalibrierung des Gasmessgerätes ein Warnsignal nach außen ausgegeben werden.

Gemäß einer bevorzugten Weiterbildung der vorliegenden Erfindung wird das Ausgabegerät abhängig von einer Ausleihrate eines Nutzers ausgegeben. So kann beispielsweise eingestellt werden, dass an einen bestimmten Nutzer nur ein mobiles Gerät pro Tag ausgegeben werden darf. Dadurch kann beispielsweise verhindert werden, dass an Nutzer, welche nicht mit ausreichender Vorsicht mit den ausgeliehenen Geräten umgehen oder dazu tendieren, ausgegebene mobile Geräte zu verlegen, mobile Geräte beliebig oft ausgegeben werden.

Gemäß einem weiteren Aspekt der vorliegenden Erfindung wird ferner ein automatisches Verfahren zum Verwalten von mobilen Geräten in einer Lagervorrichtung mit wenigstens zwei Lagerstationen, wenigstens einer Wartungsstation, wenigstens einem Controller und wenigstens einer Erkennungsvorrichtung bereitgestellt, aufweisend die folgenden Schritte:
- Erkennen eines Rückgabewunsches für die Rückgabe eines mobilen Gerätes als Rückgabegerät mittels der Erkennungsvorrichtung,
- Aufnahme des Rückgabegerätes entweder in die wenigstens eine Wartungsstation oder in eine der wenigstens zwei Lagerstationen abhängig von einem Verhältnis wenigstens eines Wartungskennwerts des Rückgabegerätes zu einem zugehörigen Wartungsschwellenwert mittels des Controllers.

Mit diesem Verfahren können mobile Geräte vollautomatisch und autonom verwaltet bzw. gezielt aufgenommen und gelagert werden.

Unter einer Aufnahme ist im Sinne der vorliegenden Erfindung beispielsweise das Aufnehmen des Rückgabegeräts in eine Lagerstation oder eine Wartungsstation der Lagervorrichtung zu verstehen, welche zum Öffnen und/oder Einlegen eines mobilen Gerätes durch einen Nutzer freigegeben wird. Diese Aufnahme wird mittels eines Controllers ausgeführt, welcher einen entsprechenden Freigabemechanismus betätigt. Das heißt, unter einer Aufnahme wird vorliegend insbesondere eine Freigabe einer Lagerstation oder einer Wartungsstation zur Ermöglichung einer Rückgabe bzw. eines Einlegens des Rückgabegerätes in die Lagerstation oder die Wartungsstation verstanden.

Der Rückgabewunsch mittels einer Erkennungsvorrichtung kann beispielsweise mittels eines Barcode-, QR-Code- oder RFID-Scanners erkannt werden. Es ist aber auch denkbar, dass der Rückgabewunsch über einen vom Nutzer zu betätigenden Touchscreen oder dergleichen erkannt werden kann.

Mit Bezug auf den Wartungskennwert sowie den Wartungsschwellenwert gilt das zum vorstehenden Verfahren ausgeführte.

Gemäß einer Weiterbildung der vorliegenden Erfindung charakterisiert der wenigstens eine Wartungskennwert ein Funktionszustand des Rückgabegerätes und der zugehörige Wartungsschwellenwert korrespondiert mit einer Funktionsfähigkeit des Rückgabegerätes. Dadurch kann das Rückgabegerät vorteilhaft in die wenigstens eine Wartungsstation oder eine der Lagerstationen aufgenommen werden. Wenn bei der Aufnahme des Rückgabegerätes beispielsweise erkannt wird, dass dieses nicht mehr funktioniert, d.h., dass der Funktionszustand des Rückgabegerätes nicht dem erforderlichen Wartungsschwellenwert entspricht oder diesen unterschreitet, kann insbesondere vermieden werden, dass dieses nicht mehr funktionierende Rückgabegerät in eine Wartungsstation aufgenommen wird, in welcher es nicht repariert werden könnte, sondern nur einen im Vergleich zur Lagerstation wertvollen Lagerplatz einnimmt.

Ferner ist es möglich, dass der wenigstens eine Wartungskennwert eine Restlaufzeit des Rückgabegerätes charakterisiert und der zugehörige Wartungsschwellenwert mit einer minimal erforderlichen Restlaufzeit des Rückgabegerätes korrespondiert. Dadurch kann das Rückgabegerät bei Unterschreitung der minimal erforderlichen Restlaufzeit beispielsweise bevorzugt in eine Wartungsstation aufgenommen werden, in welcher das Rückgabegerät gewartet werden kann. Weist das Rückgabegerät hingegen eine ausreichende Restlaufzeit auf, kann es bevorzugt in eine Lagerstation aufgenommen werden und die Wartungsstation wird für ein ggf. später eintreffendes und zu wartendes Rückgabegerät freigehalten. Dadurch ist ein effizientes und Ressourcen sparendes Verfahren zum Verwalten der mobilen Geräte realisierbar.

Außerdem ist es erfindungsgemäß möglich, dass in einem Fall, in welchem wenigstens eine Wartungsstation frei ist, das Rückgabegerät in eine Lagerstation aufgenommen wird, wenn das Rückgabegerät nicht eines von einer Anzahl von mobilen Geräten mit den kürzesten Restlaufzeiten ist und die Anzahl der mobilen Geräte mit den kürzesten Restlaufzeiten der Anzahl der wenigstens einen freien Wartungsstation entspricht. Dadurch kann gewährleistet werden, dass die wenigstens eine Wartungsstation stets für die Anzahl von mobilen Geräten mit den kürzesten Restlaufzeiten freigehalten wird. Wenn das Rückgabegerät beispielsweise ein erstes Gasmessgerät ist und die Restlaufzeit mit einem Kalibrierungszustand bzw. Kalibrierungszeitpunkt korrespondiert, wird das erste Gasmessgerät in eine Lagerstation aufgenommen, wenn erkannt wird, dass beispielsweise nur noch zwei Wartungsstationen frei sind und sich noch ein zweites und ein drittes Gasmessgerät mit jeweils einer kürzeren Restlaufzeit als das erste Gasmessgerät in Verwendung, d.h., nicht in der Lagervorrichtung, befinden. Dadurch können die jeweiligen Restlaufzeiten der mobilen Geräte stets möglichst aktuell bzw. lang gehalten werden.

Darüber hinaus ist es gemäß der vorliegenden Erfindung möglich, dass ein Warnsignal nach außen ausgegeben wird, wenn erkannt wird, dass das Verhältnis des wenigstens einen Wartungskennwerts des Rückgabegerätes zu dem zugehörigen Wartungsschwellenwert nicht einem Soll-Verhältnis entspricht. Dadurch kann verhindert werden, dass beispielsweise nicht mehr funktionierende Rückgabegeräte sowie Rückgabegeräte, die in der wenigstens einen Wartungsvorrichtung nicht gewartet werden können, in die Lagervorrichtung aufgenommen werden und dort beispielsweise eine Lagerstation blockieren. Mit Bezug auf den Alarm und das Soll-Verhältnis gilt das vorstehend hierzu gesagte in analoger Weise.

Bei einer bevorzugten Weiterbildung der vorliegenden Erfindung ist es möglich, dass, wenn erkannt wird, dass ein Verhältnis des wenigstens einen Wartungskennwerts wenigstens eines mobilen Gerätes in einer Wartungsstation zu einem zugehörigen Wartungsschwellenwert nicht einem Soll-Verhältnis entspricht, Nutzer bei der Rückgabe eines Rückgabegerätes aufgefordert werden, das wenigstens eine mobile Gerät aus der Wartungsstation zu entnehmen und in einer Lagerstation zu platzieren. Dadurch kann das erfindungsgemäße Verfahren einem Nutzer, welcher ein mobiles Gerät erhalten bzw. aus der Lagervorrichtung entwenden möchte, nutzen, um eine Verwaltungsaktion in der Lagervorrichtung auszuführen. Somit ist es nicht oder zumindest seltener erforderlich, Verwaltungspersonal als Solches für die Lagervorrichtung bereitzustellen. Die erfindungsgemäße Aufforderung kann beispielsweise mittels einer entsprechenden Schaltung der Lagervorrichtung bzw. der Lagerstationen und/oder der wenigstens einen Wartungsstation erfolgen. So ist es beispielsweise möglich, dass in diesem Fall zunächst die entsprechende Wartungsstation freigegeben wird und der Nutzer, welcher ein mobiles Gerät beispielsweise über einen Touchscreen entleihen möchte, aufgefordert wird, das betroffene Gerät aus der entsprechenden Wartungsstation zu entwenden. Anschließend kann eine ausgewählte Lagerstation freigegeben und der Nutzer weiter aufgefordert werden, das betroffene mobile Gerät in diese Lagerstation zu setzen. Erst nachdem das betroffene Gerät in diese Lagerstation gesetzt und diese Lagerstation für eine erneute Entnahme gesperrt wurde, wird der eigentliche Ausgabevorgang gestartet bzw. für den Nutzer ausführbar.

Gemäß einem weiteren Aspekt der vorliegenden Erfindung ist eine Vorrichtung zum Verwalten von mobilen Geräten bereitgestellt, aufweisend eine Lagervorrichtung mit wenigstens zwei Lagerstationen zum Lagern eines der mobilen Geräte und wenigstens einer Wartungsstation zum Lagern und Warten eines der mobilen Geräte, eine Erkennungsvorrichtung zum Erkennen eines Ausgabewunsches für die Ausgabe eines der mobilen Geräte aus der Lagervorrichtung, und einen Controller zum Ausführen des vorstehend dargestellten Verfahrens, wobei der Controller ist konfiguriert, eine Gruppe von mobilen Geräten abhängig von einem Verhältnis wenigstens eines Wartungskennwerts der jeweiligen mobilen Geräte zu einem zugehörigen Wartungsschwellenwert zu bestimmen, eines der mobilen Geräte aus der Gruppe als Ausgabegerät auszuwählen und das ausgewählte Ausgabegeräte auszugeben.

Gemäß einem weiteren Aspekt der vorliegenden Erfindung ist eine Vorrichtung zum Verwalten von mobilen Geräten bereitgestellt, aufweisend eine Lagervorrichtung mit wenigstens zwei Lagerstationen zum Lagern eines der mobilen Geräte und wenigstens einer Wartungsstation zum Lagern und Warten eines der mobilen Geräte, eine Erkennungsvorrichtung zum Erkennen eines Rückgabewunsches für die Rückgabe eines mobilen Gerätes als Rückgabegerät, und einen Controller zum Ausführen des vorstehend dargestellten Verfahrens, wobei der Controller ist konfiguriert, das Rückgabegerät in die wenigstens eine Wartungsstation oder in eine der wenigstens zwei Lagerstationen abhängig von einem Verhältnis wenigstens eines Wartungskennwerts des Rückgabegerätes zu einem zugehörigen Wartungsschwellenwert aufzunehmen.

Mittels solcher Vorrichtungen können mobile Geräte, beispielsweise Gasmessgeräte, grundsätzlich an jedem beliebigen Ort ohne menschliche Unterstützung automatisiert verwaltet werden. Insbesondere können sie aufgenommen, gelagert und/oder ausgegeben werden.

Der Controller ist bevorzugt als zentrale Steuer- und/oder Regeleinheit ausgebildet und kann Teil einer Computereinheit sein. Die Erkennungsvorrichtung kann als Nutzerschnittstelle, beispielsweise als Touchscreen und/oder eine mit physischen Tasten zu bedienende Eingabevorrichtung bereitgestellt werden, wodurch der Benutzerwunsch beispielsweise über Fingereingabe am Touchscreen oder über die Tasten eingegeben und dabei erkannt werden kann. Die Eingabevorrichtung kann alternativ oder zusätzlich als Barcode-, QR-Code- und/oder RFID-Scanner ausgebildet sein. Bevorzugt sind zum Erkennen des Ausgabewunsches ein Touchscreen und zum Erkennen des Rückgabewunsches ein Barcode-, QR-Code- und/oder RFID-Scanner bereitgestellt. Das Ausgeben und das Aufnehmen der mobilen Geräte funktionieren bevorzugt in Verbindung mit einer mechanischen und/oder elektronischen Sperrvorrichtung, welche das ausgewählte Ausgabegerät bzw. Rückgabegerät zur Ausgabe bzw. Entnahme oder Rückgabe für einen Nutzer mechanisch freigibt bzw. sperrt.

Bei der erfindungsgemäßen Ausgabe und/oder Aufnahme kann zusätzlich eine Transportvorrichtung bereitgestellt sein. Diese ist bevorzugt automatisiert. Die Transportvorrichtung kann ausgebildet sein, das Ausgabegerät von einer Lagerstation oder einer Wartungsstation zu einem Nutzer oder zumindest in seinen Zugriffsbereich und/oder das Rückgabegerät vom Nutzer in eine ausgewählte Lagerstation oder Wartungsstation zu befördern.

Gemäß einer Weiterbildung der vorliegenden Erfindung kann ferner eine Warnsignalausgabevorrichtung bereitgestellt sein, die derart konfiguriert ist, dass sie ein Warnsignal nach außen ausgibt, wenn erkannt wird, dass das Verhältnis des wenigstens einen Wartungskennwerts eines der mobilen Geräte in einer Lagerstation oder einer Wartungsstation zu dem zugehörigen Wartungsschwellenwert nicht einem Soll-Verhältnis entspricht, oder wenn erkannt wird, dass das Verhältnis des wenigstens einen Wartungskennwerts des Rückgabegerätes zu dem zugehörigen Wartungsschwellenwert nicht einem Soll-Verhältnis entspricht. Die Warnsignalausgabevorrichtung kann zum Ausgeben eines optischen, akustischen und/oder elektronischen Warnsignals ausgestaltet sein. Dadurch kann in einem Fall, in welchem der Wartungszustand wenigstens eines der mobilen Geräte nicht dem Soll-Verhältnis entspricht, beispielsweise ein Techniker über diesen Zustand informiert werden und ein entsprechendes Warnsignal an der Lagerstation und/oder Wartungsstation ausgegeben werden. Ferner ergeben sich bei dieser Weiterbildung dieselben Vorteile, wie sie bereits zum vorstehenden Verfahren mit Bezug auf die Warnsignalausgabe dargestellt wurden.

Außerdem ist es möglich, dass die wenigstens zwei Lagerstationen und die wenigstens eine Wartungsstation jeweils als Dockingstation ausgebildet sind. Sie können jeweils eine mechanische Sperrvorrichtung für eines der mobilen Geräte aufweisen. Somit ist ein Ausgabegerät bei einer Ausgabe mechanisch entsperrbar und/oder ein Rückgabegerät nach einer Aufnahme mechanisch sperrbar. Dadurch kann eine besonders kostengünstige und platzsparende Lagervorrichtung bereitgestellt werden. Im Vergleich zu einem Lagerschrank, in welchem die wenigstens zwei Lagerstationen und die wenigstens eine Wartungsstation beispielsweise in eigenen Schließfächern bereitgestellt sind, reicht es gemäß dieser Weiterbildung aus, die Dockingstationen mit dem erforderlichen Freigabemechanismus zum mechanischen Aufnehmen und Ausgeben der mobilen Geräte zu versehen. Eine Dockingstation ist zumindest im Wesentlichen eine Aufnahmevorrichtung, durch welche die mobilen Geräte mit einem festen Netz, beispielsweise einem Stromnetz und/oder einem Gasnetz, verbindbar sind. Die mechanische Sperrvorrichtung kann beispielsweise in Form eines klauenartigen Rastmittels bereitgestellt werden. So kann die Sperrvorrichtung das mobile Gerät, beispielsweise das Gasmessgerät, nach einem Einsetzen in die Lagerstation oder die Wartungsstation zumindest teilweise umgreift und somit für die Entnahme durch einen Nutzer sperrt. Die mechanische Sperrvorrichtung stellt sicher, dass nur mobile Geräte aus der bestimmten Gruppe an die Nutzer ausgegeben werden und diese nicht aus Versehen ein nicht gewartetes und/oder nicht funktionierendes mobiles Gerät aus einer Dockingstation entnehmen.

Darüber hinaus kann es erfindungsgemäß von Vorteil sein, wenn die Lagervorrichtung als Lagerschrank ausgebildet ist und die wenigstens zwei Lagerstationen und die wenigstens eine Wartungsstation jeweils in einem verschließbaren Lagerfach bereitgestellt sind, welches bei einer Ausgabe eines Ausgabegerätes und/oder bei einer Aufnahme eines Rückgabegerätes mechanisch entsperrbar ist. Dadurch kann eine für die mobilen Geräte besonders sichere Lagervorrichtung bereitgestellt werden. In den Lagerfächern des Lagerschranks können die mobilen Geräte gut vor Umwelteinflüssen geschützt werden. Der Lagerschrank und die Lagerfächer sind auf keine bestimmte Größe oder Form beschränkt. Die Anzahl der Lagerfächer ist beliebig und kann je nach Einsatzgebiet variiert werden. Durch die verschließbaren, d.h. verriegelbaren oder absperrbaren, Lagerfächer kann sichergestellt werden, dass nur gewartete mobile Geräte bzw. mobile Geräte der bestimmten Gruppe an die Nutzer ausgegeben werden und diese nicht aus Versehen ein beispielsweise nicht gewartetes mobiles Gerät aus einer Dockingstation entwenden. Ferner ist eine zusätzliche mechanische Sperrvorrichtung in den Lagerstationen oder der wenigstens einen Wartungsstation in den verschließbaren Lagerfächern möglich.

Bei einer vorteilhaften Weiterbildung der vorliegenden Erfindung ist das Verhältnis der Anzahl von der wenigstens einen Wartungsstation zu Lagerstationen kleiner oder gleich 1:2. Das Verhältnis von der wenigstens einen Wartungsstation zu den Lagerstationen wird dabei insbesondere in Abhängigkeit von einer erwarteten Tagesnutzung, d.h., einer zu erwartenden Häufigkeit von Ausgaben und Aufnahmen der mobilen Geräte und einem vorgeschriebenen Wartungsintervall bereitgestellt. Bei Gasmessgeräten als mobile Geräte und einem Kalibrierungsintervall von beispielsweise 120 Tagen wäre bei 120 Lagerstationen und bei 240 Entleihungen, d.h., 240 Ausgaben und 240 Aufnahmen, eine einzige Wartungsstation bzw. Kalibrierungsstation ausreichend, um jedes Gasmessgerät im Kalibrierungsintervall von 120 Tagen zu kalibrieren und somit automatisch stets eine gültige Kalibrierung der jeweiligen Gasmessgeräte zu erzielen. Würde man nun bei 120 Lagerstationen 12 Wartungsstationen bereitstellen, d.h., das Verhältnis wäre 1:10 und somit kleiner als 1:2, wäre bei einer Anzahl von 1920 Entleihungen in 120 Tagen, welche noch unterhalb einer in der Praxis üblichen Anzahl von Entleihungen für einen solchen Fall liegen, jedes Gasmessgerät im Kalibrierungsintervall von 120 Tagen kalibriert, wobei die älteste Kalibrierung nur 10 Tage alt wäre. Damit könnte ferner ein optional erforderlicher Funktionstest erfüllt werden, der bei Gasmessgeräten beispielsweise alle 10 Tage erforderlich ist. Das Verhältnis von der wenigstens einen Wartungsstation zu den Lagerstationen hängt dabei stark vom Einsatzgebiet der erfindungsgemäßen Vorrichtung ab. In stark verschmutzen Gebieten, in welchen mit einem häufigen Austausch von mobilen Geräten zu rechnen ist, ist das Verhältnis entsprechend höher zu wählen, beispielsweise 1:4 bis 1:2. Das Verhältnis ist dabei nicht auf 1:2 beschränkt, sondern kann bei entsprechenden Umgebungsbedingungen und Ausleihraten auch auf deutlich über 1:2 erhöht werden.

In jedem Fall ist es durch die erfindungsgemäße Vorrichtung sowie das entsprechende Verfahren möglich, mit nur einer geringen Anzahl von Wartungsstationen eine Vielzahl von mobilen Geräten wie Gasmessgeräten zu verwalten und dabei einen gewarteten bzw. kalibrierten Zustand der mobilen Geräte bzw. Gasmessgeräte zu gewährleisten. Somit können im Vergleich zu einem System, welches ausschließlich aus Wartungsstationen besteht, erhebliche Kosten eingespart werden.

Von weiterem Vorteil ist es, wenn die Lagervorrichtung eine Anzahl von mobilen Geräten aufweist, die der Anzahl der Lagerstationen entspricht. Das heißt, in einer vollständig mit mobilen Geräten ausgestatteten Lagervorrichtung sind alle Lagerstationen mit mobilen Geräten belegt und die wenigstens eine Wartungsstation ist leer. Durch die wenigstens eine Wartungsstation als Pufferspeicher kann ein automatisierter und grundsätzlich autonomer Durchlauf gewährleistet werden. So kann beispielsweise ein autonomes Ausgeben, Lagern und Aufnehmen der mobilen Geräte erfolgen.

Weitere, die Erfindung verbessernde Maßnahmen ergeben sich aus der nachfolgenden Beschreibung zu verschiedenen Ausführungsbeispielen der Erfindung, welche in den Figuren schematisch dargestellt sind. Sämtliche aus den Ansprüchen, der Beschreibung oder der Zeichnung hervorgehende Merkmale und/oder Vorteile, einschließlich konstruktiver Einzelheiten und räumlicher Anordnungen können sowohl für sich als auch in den verschiedenen Kombinationen erfindungswesentlich sein.

Es zeigen schematisch:
- Figur 1: eine Darstellung einer erfindungsgemäßen Vorrichtung zum Verwalten von mobilen Geräten gemäß einer ersten Ausführungsform,
- Figur 2: eine Darstellung einer erfindungsgemäßen Vorrichtung zum Verwalten von mobilen Geräten gemäß einer zweiten Ausführungsform, und
- Figuren 3a bis 3d: Darstellungen zu einem erfindungsgemäßen Verfahren zum Verwalten von mobilen Geräten.

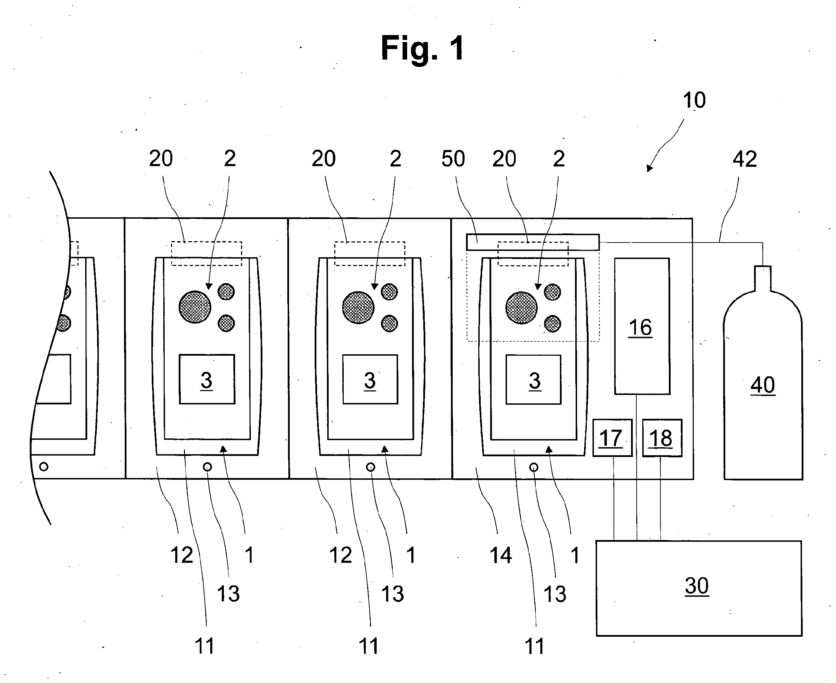

Elemente mit. gleicher Funktion und Wirkungsweise sind in den Figuren 1 bis 3d jeweils mit dem gleichen Bezugszeichen versehen.

Fig. 1 zeigt schematisch eine erfindungsgemäße Vorrichtung zum automatischen Verwalten von mobilen Geräten, genauer gesagt von Gasmessgeräten 1 gemäß einer ersten Ausführungsform der vorliegenden Erfindung. Die in Fig. 1 dargestellte Vorrichtung zum automatischen Verwalten von Gasmessgeräten 1 ist als eine Lagervorrichtung 10 mit einer Mehrzahl von zusammengeschalteten Lagerstationen 12 und einer Wartungsstation 14, die als Kalibrierstation ausgebildet ist, bereitgestellt. Die Lagerstationen 12 und die Wartungsstation 14 sind als Dockingstationen ausgebildet.

Neben der Wartungsstation 14 befindet sich eine Erkennungsvorrichtung 16, 18 bzw. eine Ausgabewunsch-Erkennungsvorrichtung in Form einer Nutzerschnittstelle, beispielsweise ein Touchscreen zum Erkennen eines Ausgabewunsches für die Ausgabe eines der Gasmessgeräte 1. Die Ausgabewunsch-Erkennungsvorrichtung 16, 18 muss dabei nicht als integrierter Bestandteil der Lagervorrichtung 10 vorgesehen sein, sondern kann auch als externe Vorrichtung bereitgestellt werden, die beispielsweise nur über ein Kabel oder kabellos, beispielsweise über eine Funkverbindung, mit der Vorrichtung zum Verwalten der Gasmessgeräten 1 in Verbindung steht. In der in Fig. 1 dargestellten Lagervorrichtung 10 ist in den Lagerstationen 12 und der Wartungsstation 14 jeweils ein Gasmessgerät 1 in einem Lagerfach 11 aufgenommen. Die Gasmessgeräte 1 weisen jeweils einen Touchscreen 3 sowie Gaseintrittsöffnungen 2 zur Aufnahme von verschiedenen Gassubstanzen auf. Die Gasmessgeräte 1 sind in den Dockingstationen jeweils mittels eines Controllers 30 der Vorrichtung zum Verwalten von Gasmessgeräten 1 durch eine mechanische Sperrvorrichtung 20 sperr- und entsperrbar. Durch die mechanische Sperrvorrichtung 20 können die Gasmessgeräte 1 wahlweise bzw. in Abhängigkeit von bestimmten Steuerparametern, beispielsweise einem Kalibrierungszustand der Gasmessgeräte 1, zur Entnahme durch einen Nutzer mechanisch freigegeben bzw. entsperrt oder gesperrt werden.

In der in Fig. 1 dargestellten Vorrichtung zum Verwalten von Gasmessgeräten 1 ist ferner eine Gaszuführleitung 42 integriert, über welche ein Prüfgas von einer Gasquelle 40 zu der Wartungsstation 14 zugeführt werden kann. Hierzu ist ferner eine schwenkbare Klappe 50 vorgesehen, welche über den freien Bereich der Gaseintrittsöffnungen 2 des jeweiligen Gasmessgerätes 1 schwenkbar ist und über welche dem jeweiligen Gasmessgerät 1 das Prüfgas zugeführt wird. Eine Lampe 13 dient zur optischen Darstellung eines durch die mechanische Sperrvorrichtung 20 freigegebenen oder gesperrten Zustands des jeweiligen Gasmessgerätes 1. Dies hat den vorteilhaften Effekt, dass ein Nutzer nicht erst versuchen muss, das Gasmessgerät 1 aus einer Dockingstation zu entwenden, sondern direkt angezeigt bekommt, ob es zur Entnahme freigegeben oder gesperrt ist.

Die Erkennungsvorrichtung 16, 18 weist ferner eine Rückgabewunsch-Erkennungsvorrichtung in Form eines Barcode-, QR-Code- oder RFID Scanners auf. Die Rückgabewunsch-Erkennungsvorrichtung muss wie die Ausgabewunsch-Erkennungsvorrichtung nicht als integraler Bestandteil der Lagervorrichtung 10 bereitgestellt werden, sondern kann auch als separates Bauteil vorgesehen sein.

Die in Fig. 1 gezeigte Vorrichtung zum Verwalten von Gasmessgeräten 1 weist ferner eine Warnsignalausgabevorrichtung 17 auf. Die Warnsignalausgabevorrichtung ist beispielsweise mit der Lampe 13 verbindbar, welche bei einem Fehler in einer bestimmten Farbe aufleuchtet.

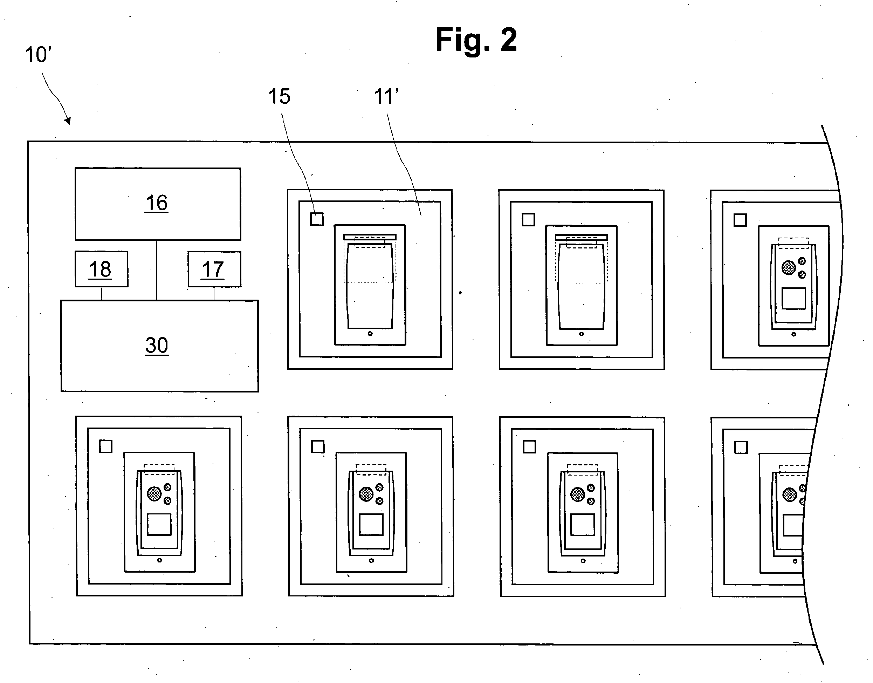

Fig. 2 zeigt eine Vorrichtung zum automatischen Verwalten von Gasmessgeräten 1 gemäß einer zweiten Ausführungsform der vorliegenden Erfindung. Zur vereinfachten Darstellung und zur Vermeidung einer mit Bezugszeichen überladenen Figur wurde in Fig. 2 teilweise auf eine Wiederholung der bereits in Fig. 1 dargestellten Bezugszeichen verzichtet. Die in Fig. 2 dargestellte zweite Ausführungsform unterscheidet sich insbesondere dadurch von der in Fig. 1' dargestellten ersten Ausführungsform, dass die Lagervorrichtung 10' als Lagerschrank bereitgestellt ist und Lagerstationen 12 und Wartungsstationen 14 in Lagerfächern 11', welche als abschließbare bzw. verriegelbare Schrankfächer ausgebildet sind, gelagert sind. Die in Fig. 2 dargestellten Lagerfächer 11' weisen ferner jeweils einen Türgriff 15 zum erleichterten Öffnen und Schließen von Lagerfachtüren auf, nachdem die mechanische Sperrvorrichtung 20 durch eine entsprechende Anweisung von dem Controller 30 entsperrt wurde. Obwohl die mechanische Sperrvorrichtung 20 in Fig. 2 nur in den Dockingstationen dargestellt ist, kann sie zusätzlich oder bevorzugt alternativ auch in den Lagerfächern 11' als Sperrmittel für die Lagerfachtüren bereitgestellt werden. Das mechanische Sperrmittel 20 ist jedoch nicht darauf beschränkt. Vielmehr ist ein beliebiges Sperrmittel vorstellbar, sofern es einen Nutzer daran hindern kann, ein Gasmessgerät 1 aus einem Lagerfach 11' bzw. einer Lagerstation 12 oder einer Wartungsstation 14 zu entwenden.

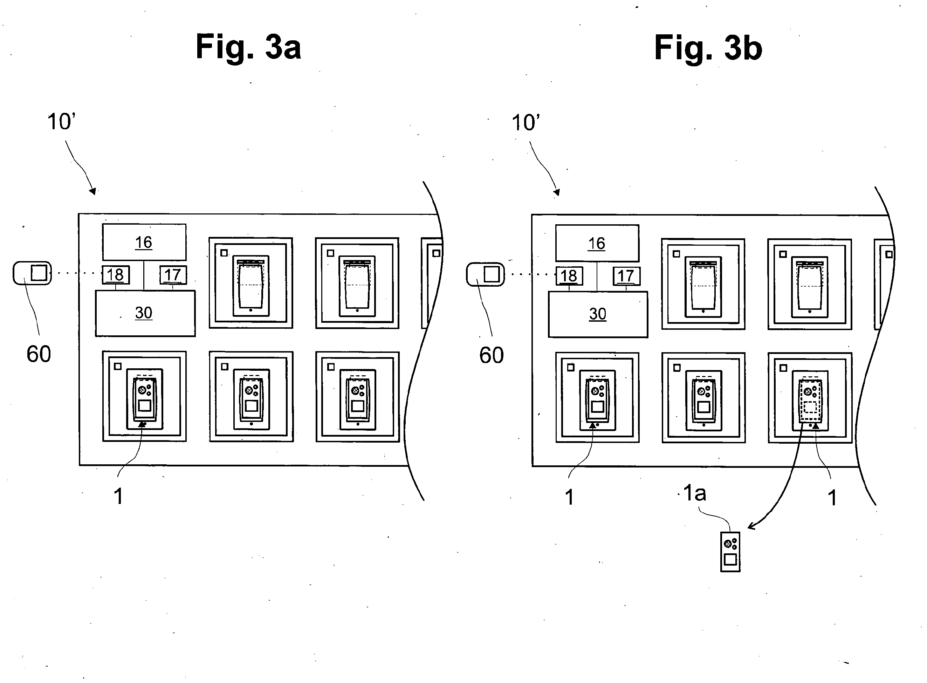

In den Figuren 3a bis 3d wird ein Verfahren zum Verwalten von mobilen Geräten bzw. Gasmessgeräten 1 in einer Lagervorrichtung 10' mit einer Mehrzahl, von Lagerstationen 12 und einer Mehrzahl von Wartungsstationen 14 dargestellt. Für eine vereinfachte Darstellung des erfindungsgemäßen Verfahrens wird in den Figuren 3a bis 3d teilweise auf die in den Figuren 1 und 2 dargestellten Bezugszeichen verzichtet.

Gemäß der Darstellung in Fig. 3a kommt ein Nutzer beispielsweise mit einer ID-Karte 60 zu der Lagervorrichtung 10' um ein Gasmessgerät 1 zu entleihen. In der in Fig. 3a dargestellten Lagervorrichtung 10' sind zwei leere Wartungsstationen 14 bereitgestellt. Die Erkennungsvorrichtung 16, 18 ist beispielsweise als Touchscreen ausgebildet. Hierbei kann im Ausgabeprozess auf einem Startbildschirm des Touchscreens ein entsprechend dargestellter Button betätigt und somit der Ausgabewunsch zur Erkennung mitgeteilt werden. Nach erfolgter Auswahl ist es ferner möglich, dass die Nutzer-ID gescannt wird, um das auszugebende Gasmessgerät 1 einem bestimmten Nutzer zuzuordnen und/oder eine Berechtigung des Nutzers zu überprüfen. Dies kann auch vor der Betätigung des Touchscreens erfolgen.

Nachdem der Ausgabewunsch des Nutzers für die Ausgabe eines Gasmessgerätes 1 erkannt wurde, wird eine Gruppe von Gasmessgeräten abhängig von einem Verhältnis wenigstens eines Wartungskennwerts, beispielsweise eines Kalibrierungszeitpunkts bzw. -zustands der jeweiligen Gasmessgeräte zu einem zugehörigen Wartungsschwellenwert bzw. Kalibrierungsschwellenwert bestimmt. Die Kalibrierungszustände können bei neuen Gasmessgeräten 1 entweder im Voraus hinterlegt sein, oder bei alten, d.h., bereits verwendeten bzw. verliehenen Gasmessgeräten 1, durch die Erfassungsvorrichtung 16, 18 erfasst und im System hinterlegt sein.

Wie in Fig. 3b gezeigt, wird nun eines der Gasmessgeräte 1 aus der bestimmten Gruppe als Ausgabegerät 1a ausgewählt und ausgegeben, d.h., für die Entnahme durch den Nutzer freigegeben. Dies geschieht beispielsweise durch Entriegeln des Lagerfachs 11'. Das in Fig. 3b dargestellte Ausgabegerät 1a ist ein funktionierendes Gasmessgerät mit einer minimal erforderlichen Restlaufzeit, bevorzugt der kürzesten Restlaufzeit. Da die beiden in Fig. 3b dargestellten Wartungsstationen 14 frei sind, wird ein Gasmessgerät 1 aus einer Lagerstation 12 ausgewählt. Hierzu kann sich beispielsweise automatisch eine Lagerfachtüre des Lagerfachs 11' mit dem Ausgabegerät 1a öffnen.

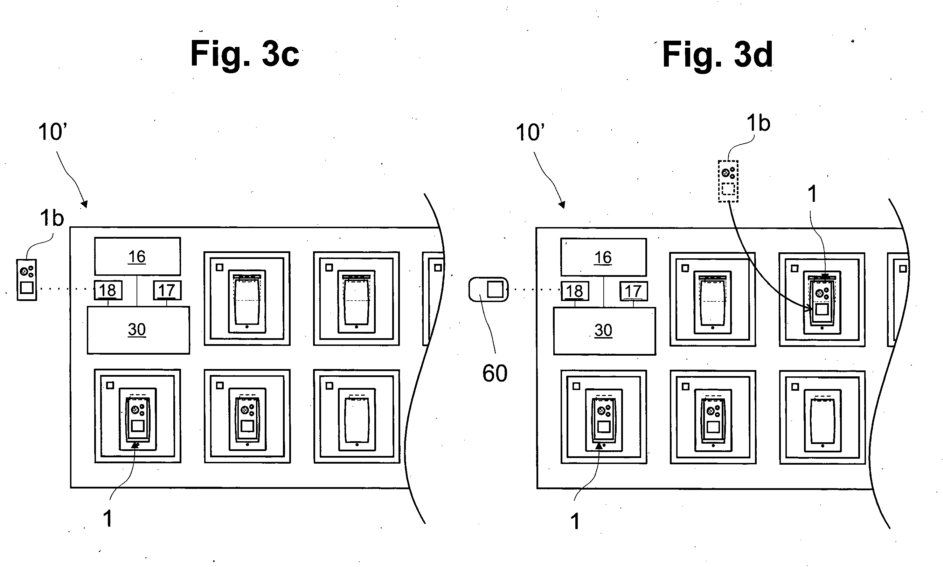

Die Rückgabe des Rückgabegerätes 1b wird in Fig. 3c und Fig. 3d dargestellt. Wie in Fig. 3c dargestellt scannt der Nutzer das Rückgabegerät 1b bei der Rückgabe zunächst über die Erkennungsvorrichtung 16, 18 ein. Alternativ wäre es auch denkbar, dass der Nutzer das Rückgabegerät 1b nicht einscannt, sondern es über den Touchscreen einträgt. Nachdem der Rückgabewunsch des Nutzers erkannt wurde, gibt die Lagervorrichtung 10' erfindungsgemäß vollautomatisch eine geeignete Lagerstation 12 oder Wartungsstation 14 zur Aufnahme des Rückgabegerätes 14 frei. Hierzu überprüft der Controller 30 zunächst, ob eine Wartungsstation 14 frei ist. Ist dies der Fall wird, wie in Fig. 3d dargestellt und unter den vorstehend beschriebenen Bedingungen, die Wartungsstation 14 und/oder das entsprechende Lagerfach 11' zur Aufnahme des Rückgabegerätes 1b freigegeben. Die Rückgabe des Rückgabegerätes 1b kann eine zusätzliche Verifizierung des Nutzers durch seine ID-Karte 60 erfordern. Sobald das Rückgabegerät 1b in der Wartungsstation 14 aufgenommen ist, kann es durch Zufuhr eines Prüfgases wieder kalibriert und zum erneuten Einsatz bereitgestellt werden. Das Rückgabegerät 1b wird dabei in der Wartungsstation 14 gelagert.

In der Wartungsstation 14 kann das Rückgabegerät 1b aufgeladen und weiter gewartet werden. Nach Ende der Wartung kann das Rückgabegerät 1b automatisch in eine freie Lagerstation 12 überführt und kann dort bis zu einer erneuten Entnahme gelagert werden, so dass die Wartungsstation 14 zur Aufnahme eines weiteren Rückgabegeräts 1b frei wird.

### Bezugszeichenliste

- 1: Gasmessgerät
- 1a: Ausgabegerät
- 1 b: Rückgabegerät
- 2: Gaseintrittsöffnungen
- 3: Touchscreen
- 10; 10': Lagervorrichtung
- 11; 11': Lagerfach
- 12: Lagerstation
- 13: Lampe
- 14: Wartungsstation
- 15: Türgriff
- 16, 18: Erkennungsvorrichtung
- 17: Warnsignalausgabevorrichtung
- 20: mechanische Sperrvorrichtung
- 30: Controller
- 40: Gasquelle
- 42: Gaszuführleitung
- 50: Klappe
- 60: ID-Karte

## Patentansprüche

1. Vorrichtung zum automatischen Verwalten von mobilen Geräten (1), aufweisend
eine als Lagerschrank ausgeführte Lagervorrichtung (10; 10') mit wenigstens zwei Lagerstationen (12) zum Lagern eines der mobilen Geräte (1) und mit wenigstens einer Wartungsstation (14) zum Lagern und Warten eines der mobilen Geräte (1) und
eine Erkennungsvorrichtung (16, 18) mit einer Nutzerschnittstelle zum Erkennen eines Ausgabewunsches für die Ausgabe eines der mobilen Geräte (1) aus der Lagervorrichtung (10; 10'),
**dadurch gekennzeichnet, dass**
die wenigstens zwei Lagerstationen (12) und die wenigstens eine Wartungsstation (14) jeweils in einem verschließbaren Lagerfach bereitgestellt sind, welches bei einer Ausgabe eines Ausgabegeräts und/oder bei einer Aufnahme eines Rückgabegeräts mechanisch entsperrbar ist,
wobei die Erkennungsvorrichtung (16, 18) einen Barcode-, QR-Code- und/oder RFID-Scanner zum Erkennen eines Rückgabewunsches für die Rückgabe eines mobilen Gerätes (1) als Rückgabegerät (1b) umfasst und
wobei die Verwaltungs-Vorrichtung einen Controller (30) umfasst, der konfiguriert ist,
eine Gruppe von mobilen Geräten (1) abhängig von einem Verhältnis wenigstens eines Wartungskennwerts der jeweiligen mobilen Geräte (1) zu einem zugehörigen Wartungsschwellenwert zu bestimmen, eines der mobilen Geräte (1) aus der Gruppe als Ausgabegerät (1a) auszuwählen, das ausgewählte Ausgabegeräte (1a) durch mechanische Entriegelung wenigstens eines der Lagerfächer auszugeben, und/oder das Rückgabegerät (1b) in die wenigstens eine Wartungsstation (14) oder in eine der wenigstens zwei Lagerstationen (12) abhängig von einem Verhältnis wenigstens eines Wartungskennwerts des Rückgabegerätes (1b) zu einem zugehörigen Wartungsschwellenwert durch Freigabe im Wege der Entriegelung wenigstens eines der Lagerfächer aufzunehmen.

2. Vorrichtung (1) nach Anspruch 1 mit einer Warnsignalausgabevorrichtung (17), die derart konfiguriert ist, dass sie ein Warnsignal nach außen ausgibt, wenn erkannt wird, dass das Verhältnis des wenigstens einen Wartungskennwerts eines der mobilen Geräte (1) in einer Lagerstation (12) oder einer Wartungsstation (14) zu dem zugehörigen Wartungsschwellenwert nicht einem Soll-Verhältnis entspricht, oder wenn erkannt wird, dass das Verhältnis des wenigstens einen Wartungskennwerts des Rückgabegerätes (1b) zu dem zugehörigen Wartungsschwellenwert nicht einem Soll-Verhältnis entspricht.

3. Vorrichtung (1) nach Anspruch 1 oder 2, wobei die wenigstens zwei Lagerstationen (12) und die wenigstens eine Wartungsstation (14) jeweils als Dockingstation ausgebildet sind und jeweils eine mechanische Sperrvorrichtung (20) für eines der mobilen Geräte (1) aufweisen, wobei ein Ausgabegerät (1a) bei einer Ausgabe mechanisch entsperrbar und/oder ein Rückgabegerät (1b) nach einer Aufnahme mechanisch sperrbar ist.

4. Vorrichtung nach einem der Ansprüche 1 bis 3, wobei das Verhältnis von der wenigstens einen Wartungsstation (14) zu Lagerstationen (12) kleiner oder gleich 1:2 ist.

5. Vorrichtung nach einem der Ansprüche 1 bis 4, wobei die Lagervorrichtung (10; 10') eine Anzahl von mobilen Geräten (1) aufweist, die der Anzahl der Lagerstationen (12) entspricht.

## Claims

1. Apparatus for automatically managing mobile devices (1), having
a storage apparatus (10; 10') which is in the form of a storage cabinet and has at least two storage stations (12) for storing one of the mobile devices (1) and has at least one maintenance station (14) for storing and maintaining one of the mobile devices (1), and
a detection apparatus (16, 18) having a user interface for detecting a desire to issue one of the mobile devices (1) from the storage apparatus (10; 10'),
**characterized in that**
the at least two storage stations (12) and the at least one maintenance station (14) are each provided in a lockable storage compartment which can be mechanically unlocked when issuing an issue device and/or when receiving a return device,
wherein the detection apparatus (16, 18) comprises a barcode, QR code and/or RFID scanner for detecting a desire to return a mobile device (1) as a return device (1b), and
wherein the management apparatus comprises a controller (30) which is configured
to determine a group of mobile devices (1) on the basis of a ratio of at least one maintenance characteristic value of the respective mobile devices (1) to an associated maintenance threshold value, to select one of the mobile devices (1) from the group as an issue device (1a), to issue the selected issue device (1a) by mechanically unlocking at least one of the storage compartments, and/or to receive the return device (1b) into the at least one maintenance station (14) or into one of the at least two storage stations (12) on the basis of a ratio of at least one maintenance characteristic value of the return device (1b) to an associated maintenance threshold value by means of approval during the unlocking of at least one of the storage compartments.

2. Apparatus (1) according to Claim 1, having a warning signal output apparatus (17) which is configured to output a warning signal to the outside if it is detected that the ratio of the at least one maintenance characteristic value of one of the mobile devices (1) in a storage station (12) or a maintenance station (14) to the associated maintenance threshold value does not correspond to a desired ratio or if it is detected that the ratio of the at least one maintenance characteristic value of the return device (1b) to the associated maintenance threshold value does not correspond to a desired ratio.

3. Apparatus (1) according to Claim 1 or 2, wherein the at least two storage stations (12) and the at least one maintenance station (14) are each in the form of a docking station and each have a mechanical locking apparatus (20) for one of the mobile devices (1), wherein an issue device (1a) can be mechanically unlocked during issue and/or a return device (1b) can be mechanically locked after reception.

4. Apparatus according to one of Claims 1 to 3, wherein the ratio of the at least one maintenance station (14) to storage stations (12) is less than or equal to 1:2.

5. Apparatus according to one of Claims 1 to 4, wherein the storage apparatus (10; 10') has a number of mobile devices (1) corresponding to the number of storage stations (12).

## Revendications

1. Dispositif pour la gestion automatique d'appareils mobiles (1), présentant
un dispositif de stockage (10 ; 10') réalisé sous la forme d'une armoire de stockage avec au moins deux stations de stockage (12) pour le stockage d'un des appareils mobiles (1) et avec au moins une station de maintenance (14) pour le stockage et la maintenance d'un des appareils mobiles (1) et
un dispositif de détection (16, 18) avec une interface utilisateur pour détecter une demande de sortie pour la sortie d'un des appareils mobiles (1) du dispositif de stockage (10 ; 10'),
**caractérisé en ce que**
lesdites au moins deux stations de stockage (12) et ladite au moins une station de maintenance (14) sont prévues chacune dans un compartiment de stockage verrouillable qui peut être déverrouillé mécaniquement lors d'une sortie d'un appareil de sortie et/ou lors d'une réception d'un appareil de retour,
le dispositif de détection (16, 18) comprenant un scanner de code à barres, de code QR et/ou de RFID pour détecter une demande de retour pour le retour d'un appareil mobile (1) en tant qu'appareil de retour (1b) et
le dispositif de gestion comprenant un contrôleur (30) qui est configuré pour déterminer un groupe d'appareils mobiles (1) en fonction d'un rapport entre au moins une valeur caractéristique de maintenance des appareils mobiles respectifs (1) et une valeur seuil de maintenance associée, sélectionner un des appareils mobiles (1) du groupe comme appareil de sortie (1a), effectuer la sortie de l'appareil de sortie (1a) sélectionné en déverrouillant mécaniquement au moins un des compartiments de stockage et/ou pour recevoir l'appareil de retour (1b) dans ladite au moins une station de maintenance (14) ou dans l'un desdits au moins deux compartiments de stockage (12) en fonction d'un rapport entre au moins une valeur caractéristique de maintenance de l'appareil de retour (1b) et une valeur seuil de maintenance associée par libération en déverrouillant au moins un des compartiments de stockage.

2. Dispositif (1) selon la revendication 1, avec un dispositif d'émission de signal d'avertissement (17) qui est configuré de telle sorte qu'il émet un signal d'avertissement vers l'extérieur lorsqu'il est détecté que le rapport entre ladite au moins une valeur caractéristique de maintenance d'un des appareils mobiles (1) dans une station de stockage (12) ou une station de maintenance (14) et la valeur seuil de maintenance correspondante ne correspond pas à un rapport de consigne ou lorsqu'il est détecté que le rapport entre ladite au moins une valeur caractéristique de maintenance de l'appareil de retour (1b) et la valeur seuil de maintenance correspondante ne correspond pas à un rapport de consigne.

3. Dispositif (1) selon la revendication 1 ou 2, dans lequel lesdites au moins deux stations de stockage (12) et ladite au moins une station de maintenance (14) sont chacune conçues comme une station d'accueil et présentent chacune un dispositif de verrouillage mécanique (20) pour un des appareils mobiles (1), dans lequel un appareil de sortie (1a) peut être déverrouillé mécaniquement lors d'une sortie et/ou un appareil de retour (1b) peut être verrouillé mécaniquement après une réception.

4. Dispositif selon l'une des revendications 1 à 3, dans lequel le rapport entre ladite au moins une station de maintenance (14) et les stations de stockage (12) est inférieur ou égal à 1:2.

5. Dispositif selon l'une des revendications 1 à 4, dans lequel le dispositif de stockage (10 ; 10') présente un nombre d'appareils mobiles (1) qui correspond au nombre de stations de stockage (12).
